# EUROPÄISCHE PATENTSCHRIFT

(11) **EP 0 879 022 B1**
(45) Veröffentlichungstag und Bekanntmachung des Hinweises auf die Patenterteilung: **30.08.2006**
(21) Anmeldenummer: 96938183.9
(22) Anmeldetag: 12.11.1996
(51) Int. Cl.: A61C 3/00, A61B 17/00, A63H 33/00, A63H 3/00

(54) **BEHANDLUNGSINSTRUMENT FÜR DIE MEDIZINISCHE ODER ZAHNMEDIZINISCHE BEHANDLUNG VON KINDERN**
INSTRUMENT FOR THE MEDICAL OR DENTAL TREATMENT OF CHILDREN
INSTRUMENT POUR LE TRAITEMENT MEDICAL OU DENTAIRE PEDIATRIQUE

(43) Veröffentlichungstag der Anmeldung: 25.11.1998
(73) Patentinhaber: Simoes, Dionisio Rio, 8200 Albufeira (PT)
(72) Erfinder: Simoes, Dionisio Rio, 8200 Albufeira (PT)
(74) Vertreter: Dreiss, Fuhlendorf, Steimle & Becker
(86) Internationale Anmeldenummer: PCT/EP1996/004945
(87) Internationale Veröffentlichungsnummer: WO 1998/020804

(56) Entgegenhaltungen:
- WO-A-94/25082
- DE-A- 3 236 618
- DE-U- 8 418 590
- DE-U- 8 900 354
- US-A- 3 299 891

## Beschreibung

Die Erfindung betrifft ein Behandlungsinstrument für die medizinische oder zahnmedizinische Behandlung von Kindern mit einem Behandlungsteil, welches ein Werkzeug und einen daran anschließenden Griff aufweist.

Vielen Kindern jagt die ärztliche Behandlung als solche und vor allem die chromblitzenden ärztlichen Instrumente nicht selten einen solchen Schrecken ein, daß sie jede Mitarbeit bei einer Heilbehandlung oder bei der Vorbeugung von Erkrankungen verweigern. Das macht einerseits für den Arzt den Umgang mit seinen kleinen Patienten schwierig. Andererseits fällt es dann auch schwer, die Kinder an ärztliche Behandlung oder vorbeugende Maßnahmen zur Gesundheitsvorsorge im privaten Bereich heranzuführen. Ärzte und Eltern müssen daher auch pädagogische Maßnahmen ergreifen, um Kindern diese Scheu zu nehmen.

Es sind verschiedene Vorrichtungen bekannt, die mit einem zur Ablenkung dienenden Element versehen sind und somit speziell zur Behandlung von Kindern eingesetzt werden.

Ein gattungsgemäßes Behandlungsinstrument ist aus DE 8900354U bekannt. Dabei handelt es sich um ein Instrument für die medizinische Behandlung von Kindern mit einem Behandlungsteil, welches ein Werkzeug (die Pinzetten), einen daran anschließenden Griff und ein darauf fest montiertes Spielteil aufweist. Dieses Spielteil ist figürlich ausgestaltet und kann als Sichtblende dienen, die angsthemmend wirken soll.

DE 32 36 618 A offenbart eine Zahnbürste, insbesondere für Kinder, an deren freien Ende des Griffs eine lösbar gehaltene Figur montiert ist.

Aus US-A-3 299 891 ist eine Spritze bekannt, deren Griff figürlich ausgestaltet ist. Die Figur ist lösbar befestigt und nach der Behandlung als Spielzeug verwendbar.

WO 94 25082 A zeigt eine Vorrichtung zum Absaugen von Nasenschleim, auf der ein Spielteil montiert ist, das gleichzeitig als Funktionsteil eingesetzt wird.

Aufgabe der Erfindung ist es daher, eine Vorrichtung der o.g. Art derart weiterzubilden, dass die Ablenkung der Kinder während der Behandlung besser wird.

Die Lösung besteht darin, daß das Behandlungsinstrument am freien Ende des Griffs ein bewegliches Spielteil mit mindestens einem Spielgegenstand aufweist.

Mit dem erfindungsgemäßen "kinderfreundlich gestalteten Behandlungsinstrument" werden die Kinder einerseits in verstärktem Maß dadurch spielerisch abgelenkt, dass das Spielteil - angeregt durch die ärztliche Handhabung des Instruments - hin und her wippt, so dass die Ablenkung sehr viel stärker als bei feststehenden Spielteilen gegeben ist. Die kindliche Scheu vor der ärztlichen Behandlung wird also reduziert. Indem den Kindern ihre Angst genommen wird, erfüllt der erfindungsgemäße Gegenstand gleichzeitig einen pädagogischen Zweck, da die Kinder an Heilbehandlungen und Gesundheitsvorsorge herangeführt werden, ohne daß Angst und Ablehnung überhaupt erst entstehen.

Vorteilhafterweise kann man das Spielteil vom Spielgegenstand und/oder vom Griff des Behandlungsteils abnehmen. Damit können beide Teile getrennt hergestellt und frei miteinander kombiniert werden.

Für die Handhabbarkeit des erfindungsgemäßen Behandlungsinstrumentes ist es von Vorteil, wenn das Spielteil über ein Verlängerungsteil mit dem Griff des Behandlungsteils verbunden ist, denn dies gewährt dem Arzt eine größere Bewegungsfreiheit, so daß das zusätzlich angebrachte Spielteil bei der Behandlung nicht stört. Demselben zweck dient ein elastisches Element, z.B. in Form einer Feder, am Spielteil bzw. am Spielgegenstand. Außerdem lenkt ein wippendes, sich bewegendes Spielzeug die Kinder noch stärker ab und ermutigt sie vielleicht sogar zum Mitspielen, ohne daß der behandelnde Arzt seine Behandlung jedes Mal unterbrechen muß.

Welcher Art das Spielzeug ist, ist völlig freigestellt und hängt von den persönlichen Vorlieben, aber auch von der Handhabbarkeit ab, so daß etwa bei der Größe des Spielteils eine Obergrenze anzunehmen ist. Ansonsten ist man gestalterisch frei; man kann z.B. auf Tierdarstellungen, Märchenfiguren, Comic- oder Trickfilmfiguren zurückgreifen, deren Beliebtheit bei Kindern ja auch der Mode unterworfen ist.

Aus hygienischen Gründen sind die Teile des Behandlungsinstruments vorzugsweise aus Kunststoff oder Hartgummi gefertigt, wobei beim Einsatz des Materials keine Beschränkungen aufzuerlegen sind. Welcher Kunststoff bzw. welches Hartgummi verwendet wird, hängt primär vom Einsatz des Behandlungsinstruments ab, d.h. wie hart und widerstandsfähig oder wie flexibel es sein muß. Behandlungsinstrumente aus Kunststoff oder Hartgummi sind meist Einwegmaterialien, die anschließend weggeworfen werden. Dabei hat die Verwendung von Hartgummi den Vorteil, daß die Entsorgung aus ökologischen Gesichtspunkten einfacher ist. Das eigentliche Behandlungsteil bzw. das Verlängerungsteil können aber auch aus Metall gefertigt sein, so daß sie sterilisierbar und wiederverwendbar sind. Dabei zeigt sich ein weiterer besonderer Vorteil der Mehrteiligkeit des erfindungsgemäßen Behandlungsinstruments, d.h. die Tatsache, daß das Spielteil nach jeder Behandlung vom eigentlichen Behandlungsteil getrennt und an einem neuen Behandlungsteil wieder befestigt wird. Man kann das erfindungsgemäße Behandlungsinstrument so zusammenstecken, wie man es gerade braucht und somit jeder Behandlungsart - und auch den Vorlieben der Kinder - anpassen.
Je nach Ausgestaltung können aber auch alle Teile, also auch das Spielteil, als Wegwerfartikel gedacht sein.

Das erfindungsgemäße Behandlungsinstrument ist grundsätzlich für alle medizinischen oder zahnmedizinischen Behandlungen von Kindern geeignet. Es bietet sich besonders für solche Instrumente an, die selber relativ klein sind und von den Kindern bewußt als angsteinflössend wahrgenommen werden. Beispiele sind in der Zahnmedizin die zahlreichen zahnärztlichen Instrumente, aber auch solche Instrumente, die beim Augenarzt oder beim Hals-Nasen-Ohrenarzt oder beim Hausarzt Verwendung finden.

Ein Ausführungsbeispiel der vorliegenden Erfindung wird im folgenden anhand der beigefügten Zeichnungen näher beschrieben. Es zeigen:

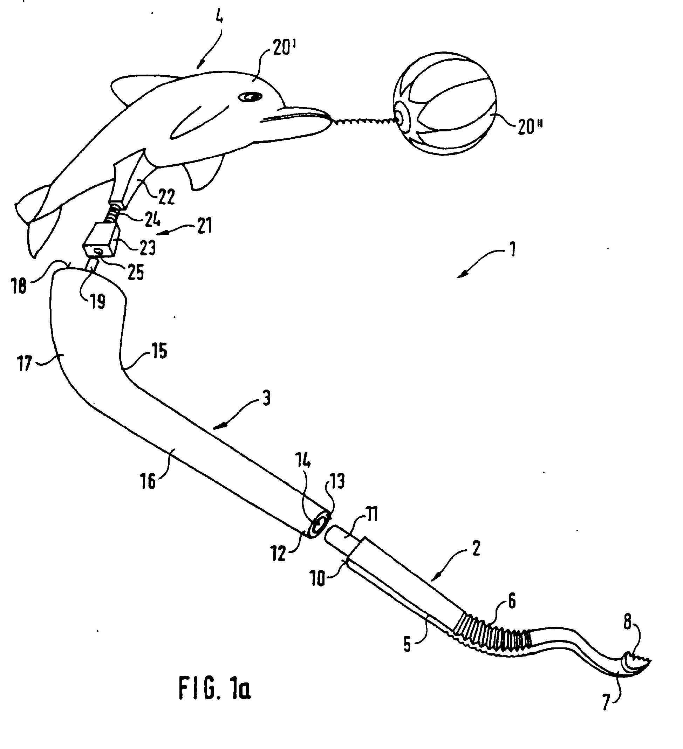

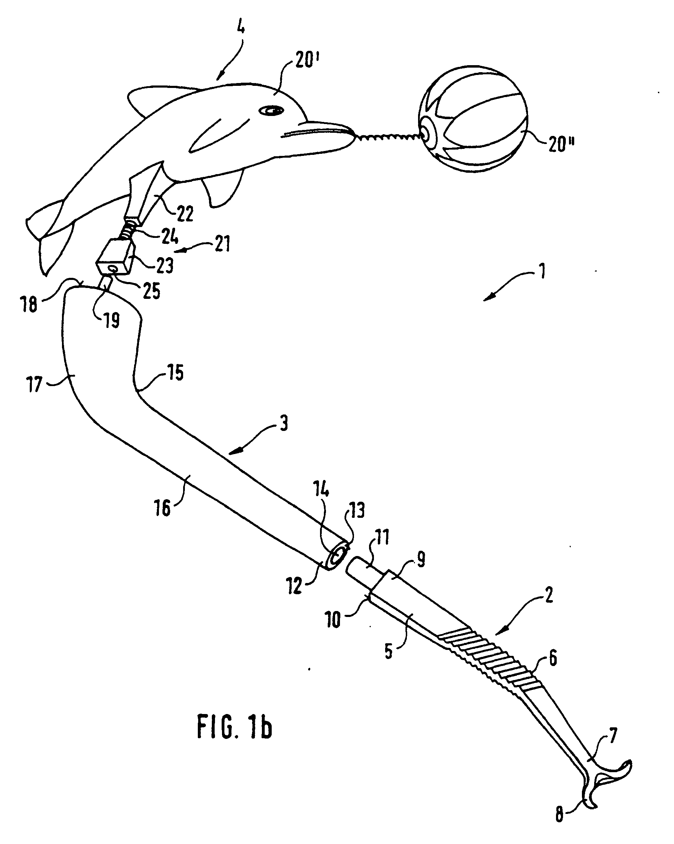

- Figur 1: ein erfindungsgemäßes Behandlungsinstrument in auseinandergezogener Darstellung;
- Figuren 2a bis 2e: beispielhaft einige Behandlungsteile für die zahnmedizinische Behandlung von Kindern.

Die vorliegende Erfindung wird im folgenden am Beispiel von zahnmedizinischen Behandlungsinstrumenten für Kinder beschrieben. Dies soll aber keine Beschränkung auf solche Instrumente darstellen. Vielmehr können Behandlungsinstrumente aller Art gemäß der vorliegenden Erfindung gestaltet werden.

Das erfindungsgemäße Behandlungsinstrument 1 ist im Ausführungsbeispiel dreiteilig. Es weist neben dem eigentlichen Behandlungsteil 2 ein Verlängerungsteil 3 und ein Spielteil 4 auf. Dabei ist das Verlängerungsteil 3 kein zwingend notwendiger Bestandteil, aber vorteilhaft, um das Spielteil 4 in einem größeren Abstand zum Behandlungsteil 2 zu bringen, so daß der Arzt bei seiner Arbeit nicht behindert wird.

Das Behandlungsteil 2 besteht im wesentlichen aus einem gebogenen oder geknickten Griff mit einer Riffelung 6 zur besseren Handhabung. Der Griff 5 läuft an einem Ende 7 in das eigentliche Werkzeug 8, hier zur zahnärztlichen Behandlung aus. Dabei verjüngt sich der Griff 5 zum Werkzeug 8 hin. Das somit etwas dickere freie Ende 9 des Griffs 5 weist eine flache Stirnfläche 10 auf, auf der mittig ein zylinderförmiger Aufsatz 11 angebracht ist. Der Aufsatz 11 ist in diesem Fall einstückig mit dem Griff 5.

Auf das Behandlungsteil 2 wird das Verlängerungsteil 3 aufgesteckt. Das Verlängerungsteil 3 ist an seinem unteren freien Ende 12 im Querschnitt kreisförmig. Das freie Ende 12 bildet eine Stirnfläche 13, die eine Bohrung 14 aufweist, deren Durchmesser so bemessen ist, daß der Aufsatz 11 am freien Ende 9 des Griffs 5 des Behandlungsteils 2 genau hineinpaßt und klemmend gehalten wird. Der Durchmesser des Verlängerungsteils 3 vergrößert sich geringfügig von seinem unteren freien Ende 12 bis zu einem Knick 15, an dem es in einem Winkel von etwa 40° zur Seite abknickt. Dadurch entstehen ein langer im Querschnitt kreisförmiger Schenkel 16 und ein kurzer Schenkel 17. Der kurze Schenkel 17 weist gegenüber dem langen Schenkel 16 einen wesentlich größeren Durchmesser auf und ist im Querschnitt nicht mehr kreisförmig, sondern oval. Er schließt mit einer Stirnfläche 18 ab, auf der wiederum ein Aufsatz 19 ausgebildet ist. Dieser Aufsatz kann, wie im vorliegenden Ausführungsbeispiel, ebenfalls zylinderförmig sein, er kann mittig oder außermittig angeordnet sein und verschiedene Durchmesser aufweisen. Bevorzugt ist jedoch eine außermittige Anordnung und die Ausbildung als relativ dünner Stift, wie im Ausführungsbeispiel gezeigt. Das hat den Vorteil, daß das auf den Aufsatz 19 aufzusetzende Spielteil 4 etwas seitlich zur Achse des kurzen Schenkels 17 angeordnet ist, so daß es bei der Behandlung dem behandelnden Arzt weniger im Weg ist.

Das Spielteil 4 wird wiederum auf den Aufsatz 19 des Griffteils 3 aufgesetzt. Im vorliegenden Ausführungsbeispiel ist das Spielteil 4 ein Spielgegenstand 20 in Form eines Delphins 20' und eines Balles 20". Am Spielgegenstand 20, im Ausführungsbeispiel an der Bauchseite des Delphins 20', ist ein Anschlußstück 21 angebracht, das einen spielzeugseitigen oberen Bereich 22 und einen instrumentenseitigen unteren Bereich 23 aufweist. Die Bereiche 22 und 23 sind durch eine Spiralfeder 24 miteinander verbunden, so daß dem Spielteil 4 eine gewisse Elastizität und Beweglichkeit gegenüber den Bewegungen des Behandlungsinstrumentes 1 in der Hand des Arztes und eventuell gegenüber dem Zugriff des Kindes gegeben ist. Der untere Bereich 23 des Anschlußstücks 21 ist mit einer Bohrung 25 versehen, deren Durchmesser wiederum dem Aufsatz 19 des Verlängerungsteils 3 angepaßt ist, so daß das Anschlußstück 21 aufgesteckt werden kann und klemmend gehalten ist.

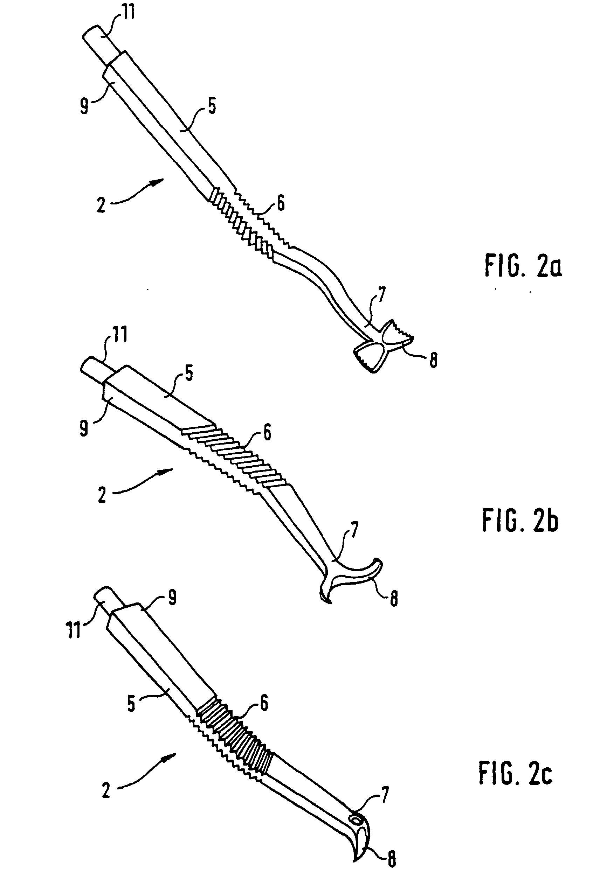

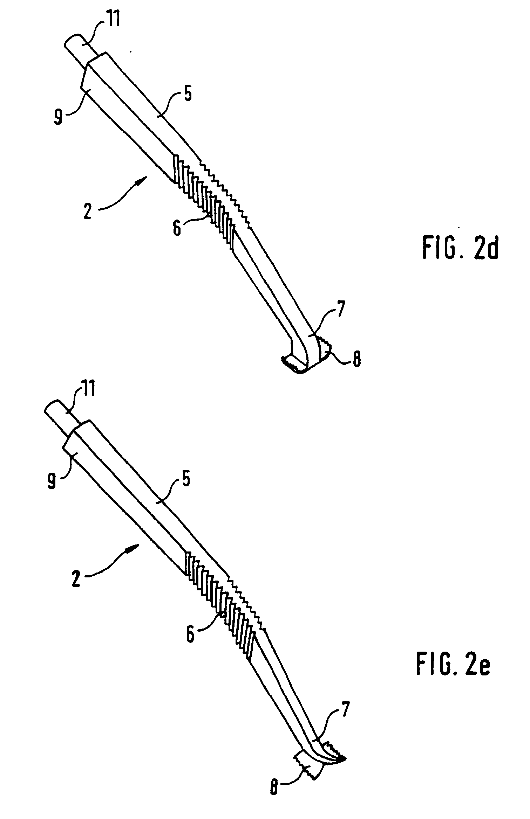

In den Figuren 2a bis 2e sind lediglich beispielhaft einige zahnärztliche Instrumente für Kinder dargestellt, die erfindungsgemäß als Behandlungsteil 2 für das Behandlungsinstrument ausgebildet sind. Das in Figur 2a gezeigte Behandlungsteil dient zur Behandlung der Zähne 1 und 2 im Oberkiefer und Unterkiefer bei Kindern der Altersgruppe von 6 bis 7 Jahren. Das in Figur 2b gezeigte Behandlungsteil dient zur Behandlung der Zähne 4, 5 und 6 im Unterkiefer von Kindern in der Altersgruppe von 9 bis 10 Jahren. Das in Figur 2c gezeigte Behandlungsteil dient zur Entfernung von Wurzelresten im Oberkiefer und Unterkiefer, während die in den Figuren 2d und 2e gezeigten Behandlungsteile zur Behandlung der Backenzähne von Kindern in der Altersgruppe von 9 bis 10 Jahren dienen.

All diesen Behandlungsteilen ist die Ausbildung in Form eines Griffes 5 gemeinsam, der ergonomisch geformt und mit einer Riffelung 6 versehen ist. An dem einen Ende 7 läuft der Griff 5 in das eigentliche Werkzeug 8 aus und an seinem freien Ende 9 endet der Griff in einer Stirnfläche 10, die den Aufsatz 11 für das Aufstecken des Verlängerungsteils 3 aufweist.

## Patentansprüche

1. Behandlungsinstrument für die medizinische oder zahnmedizinische Behandlung von Kindern mit einem Instrument (2) bestehend aus einem Werkzeug(8), einem daran anschließenden Griff (5) und einer am freien Ende des Griffs (5) aufsteckbaren Figur, **dadurch gekennzeichnet, dass** das freie Ende des Griffs (5) derart ausgestaltet ist, dass darauf ein Verlängerungsteil (3) mit seinem einen Ende aufsteckbar ist, dass ein mit dem Verlängerungsteil (3) an seinem anderen Ende verbundenes und ein elastisches Element (24) enthaltendes Anschlussstück (21) vorgesehen ist und dass die Figur ein mit dem Anschlussstück (21) verbundenen Spielteil (4, 20) ist.

2. Behandlungsinstrument nach Anspruch 1, **dadurch gekennzeichnet, dass** das elastische Element (24) mit dem Spielteil (4) einstückig ist.

3. Behandlungsinstrument nach Anspruch 1 oder 2, **dadurch gekennzeichnet, dass** das Anschlussstück (21) klemmend auf das Verlängerungsteil (3) aufgesteckt ist.

4. Behandlungsinstrument nach einem der vorhergehenden Ansprüche, **dadurch gekennzeichnet, dass** das Instrument (2) und/oder das Verlängerungsteil (3) und/oder das Spielzeug (4) aus Kunststoff oder Hartgummi gefertigt ist.

5. Behandlungsinstrument nach einem der vorhergehenden Ansprüche, **dadurch gekennzeichnet, dass** das Instrument (2) der Behandlung oder Diagnose von Kindern im Bereich der Zahnheilkunde der Hals-Nasen-Ohren-Heilkunde, der Augenheilkunde oder der allgemeinärztlichen Behandlung dient.

## Claims

1. Instrument for the medical or dental treatment of children with an instrument (2), including a tool (8) and a handle (5) adjoining the tool, and a figure, which is capable of being mounted to the handle (5), **characterized in that** the free end of the handle (5) is formed in such a manner, that an extension member (3) can be mounted with its free end thereon, and that a connecting member (21) is connected to the other end of the extension member (3), comprising an elastic element (24), and **in that** the figure is a play device (4, 20) connected to the connecting member (21).

2. Instrument in accordance to claim 1, **characterized in that** the elastic element (24) and the play device (4) are made as one piece.

3. Instrument in accordance to claim 1 or 2, **characterized in that** the connecting member (21) is mounted to the extension member (3) by being clamped thereon.

4. Instrument in accordance to one of the preceding claims, **characterized in that** the instrument (2) and/or the extension member (3) and/or the play device (4) is made of plastic material or hard rubber.

5. Instrument in accordance to one of the preceding claims, **characterized in that** the instrument (2) serves for the treatment or diagnosis of children in the field of dentistry, throat, nose, and ear medicine, ophthalmology, and general medicine.

## Revendications

1. Instrument de traitement pour le traitement médical ou dentaire des enfants avec un instrument (2) se composant d'un outil (8), un prise (5) et un chiffre pour mettre à la fin libre de la prise (5) marqué par, que la fin libre de la prise (5) est developée de telle manière, que on peut mettre une partie de prolongation avec sa fin d'un coté (3), qu'avec la partie de prolongation (3) à son autre fin liée un raccord (21) qui contient un élément flexible (24) ou on peut accoupler un jouet (figure) (4, 20).

2. Instrument de traitement vers l'exigence 1, **caractérisé** du fait, que l'élément (24) flexible avec le jouet est (4) une unique pièce.

3. Instrument de traitement vers l'exigence 1 ou 2 **caractérisé** du fait, que le raccord (21) est attaché sur la partie de prolongation (3).

4. Instrument de traitement vers une des exigences antérieures **caractérisé** du fait, que l'instrument (2) et/ou la partie de prolongation (3) et/ou le jouet (4) sont fabriqué de matière plastique ou de caoutchouc dure.

5. Instrument de traitement vers une des exigences antérieures **caractérisé** du fait, que l'instrument (2) est utilisable pour la diagnostic des enfants dans l'aire de traitement dentaire, oto-rhino-laryngologie, ophtalmologie, et dans les traitements de la médicine générale.
